# EUROPEAN PATENT APPLICATION

(11) **EP 3 831 405 A1**
(43) Date of publication of application: **09.06.2021**
(21) Application number: 19844097.6
(22) Date of filing: 02.08.2019
(51) Int. Cl.: A61K 39/085, A61K 9/06, A61K 47/36, A61P 15/14

(54) **MUCOSAL VACCINE COMPOSITION FOR BOVINE MASTITIS**

(30) Priority: 03.08.2018 JP 2018147175
(71) Applicant: NATIONAL AGRICULTURE AND FOOD RESEARCH ORGANIZATION, Tsukuba-shi, Ibaraki 305-8517 (JP); Kyoto University, Kyoto-shi, Kyoto 606-8501 (JP); Tohoku University, Sendai-shi, Miyagi 980-8577 (JP)
(72) Inventor: HAYASHI Tomohito, Sapporo-shi, Hokkaido 062-0045 (JP); KIKU Yoshio, Sapporo-shi, Hokkaido 062-0045 (JP); NAGASAWA Yuya, Sapporo-shi, Hokkaido 062-0045 (JP); AKIYOSHI Kazunari, Kyoto-shi, Kyoto 606-8501 (JP); SAWADA Shin-ichi, Kyoto-shi, Kyoto 606-8501 (JP); ASO Hisashi, Sendai-shi, Miyagi 980-8577 (JP); NOCHI Tomonori, Sendai-shi, Miyagi 980-8577 (JP)
(74) Representative: J A Kemp LLP
(86) International application number: PCT/JP2019/030451
(87) International publication number: WO 2020/027318

(57) **Abstract**

A mucosal vaccine composition for preventing bovine mastitis, comprising: an antigen derived from Staphylococcus aureus; and a nanogel comprising a polysaccharide having a cationic functional group and a hydrophobic functional group in side chains. By administering the composition to cattle, it is possible to enhance the titer of an antibody against Staphylococcus aureus immediately after the contact with the bacteria in the udder, and prevent the bovine mastitis.

## Description

### [Technical Field]

The present invention relates to a mucosal vaccine composition used for preventing bovine mastitis.

### [Background Art]

Mastitis (mammary gland inflammation) is an infectious disease in which bacteria enter the mammary gland through openings in the nipple of the udder, colonize in the mammary gland, and then repeat proliferation to damage mammary cells and cause inflammation.

Mastitis of cows occurs at milk production sites in all the countries across the world including Japan, and has a high incidence. Milk from cows suffering from mastitis is low in quality and quantity. In addition, since treatment using antibiotics is generally conducted against mastitis, milk in which an antibiotic remains during the period of treatment cannot be shipped out. Moreover, in the case where treatment using antibiotics is ineffective, cows with mastitis are dealt with as worthless cows as having low profitability and could eventually be discarded. For this reason, mastitis is a major cause of economic loss in the dairy industry, and there is a strong demand for the development of a method for preventing mastitis.

Mastitis is a complex disease that is caused by various bacterial species, and among these bacterial species, Staphylococcus aureus (S. aureus) is most common. In view of this, for preventing mastitis and other purposes, vaccines targeting this bacterium were developed (PTL 1).

In addition, vaccines targeting Staphylococcus aureus have already been commercially available such as STARTVAC (Registered Trademark, HIPRA), Lysigin (Registered Trademark, Boehringer Ingelheim), and the like.

However, many of these intramuscular injection vaccines are for increasing the antibody titer of IgG in serums and do not increase the antibody titers of antibodies (such as IgA) other than IgG in the mammary glands which contribute to the bacterial infection prevention, and also cannot sufficiently induce cellular immune responses.

In addition, Staphylococcus aureus enters mammary gland epithelial cells in a very early stage, and as a result, the bacterium becomes unlikely to be immunologically attacked by antibodies.

Hence, there is a demand for the development of a vaccine that can enhance the antibody titers of IgA and the like immediately after the contact with Staphylococcus aureus in the mammary gland to effectively suppress the infection of the bacterium.

### [Citation List]

### [Patent Literatures]

[PTL 1] Japanese Unexamined Patent Application Publication No. 2009-286730
[PTL 2] Japanese Unexamined Patent Application Publication No. 2015-151375

### [Non Patent Literatures]

[NPL 1] Boerhout E. et al., Vet Res., 2015, September 28; 46: 115

### [Summary of Invention]

### [Technical Problem]

The present invention has been made in view of the above-described problem of the conventional techniques, and an object of the present invention is to provide a vaccine composition that can enhance the titers of antibodies (IgA and the like) against Staphylococcus aureus immediately after the contact with the bacteria in the udder (the mammary gland, and milk secreted from the mammary gland, and the like) to prevent mastitis.

### [Solution to Problem]

The present inventors focused on mucosal vaccines in order to achieve the above-described object. Mucous membranes induce immune reaction on the surfaces thereof and function as an initial important defense line against pathogens such as bacteria. For example, in order to inhibit the entry of a pathogen into the body and inactivate the pathogen, mucosa secretes antibodies such as IgA against the pathogen. In addition, production cells of IgA and the like induced in a certain mucosal tissue propagate to another distant mucosal tissue, triggering the mucosal immune reaction specific to the pathogen of interest in the distant tissue.

The mucosal vaccine utilizes such propagation of immune reaction between mucosal tissues, and the present inventors made the concept of triggering the immune reaction against Staphylococcus aureus in the mucosal tissue of the bovine mammary gland by using the vaccine.

In addition, when an antigen is simply administered to mucosa, immunogenicity is generally low. For this reason, in order for the mucosal vaccine to function, a carrier (vector, delivery system, or the like) and a powerful adjuvant are required. In view of this, the present inventors focused on a cationic nanogel as a carrier for a vaccine that triggers immune reaction in the mucosal tissue of the bovine mammary gland.

Regarding the cationic nanogel, the present inventors succeeded in inducing the immunological defense mechanism against the infection of Streptococcus pneumoniae by nasally administering, to rodents and primates, a vaccine containing a complex of a nanogel (cCHP nanogel) that was obtained by adding hydrophobic cholesterol and an amino group to a pullulan as side chains and the surface protein (PspA) of Streptococcus pneumoniae (PTL 2). However, it has not been clear whether this vaccine is effective in ruminants such as cattle, and particularly whether this vaccine can trigger immune reaction in the mucosal tissue of the mammary gland.

Moreover, it was previously reported that nebulization a vaccine containing an oil-in-water emulsifier, alginate hydrogel, and antigenic proteins (Efb and LukM) derived from Staphylococcus aureus into the nasal cavity with a spray did not affect the antigenic protein-specific IgA production level in not only milk but also nasal discharge; hence, it has been suggested that not only the above-described intramuscular injection vaccine but also the mucosal vaccine cannot increase the antibody titers of IgA and the like which contribute to the bacterial infection prevention and cannot sufficiently induce the cellular immune response in the bovine mammary gland (NPL 1).

In view of this, we prepared a complex of Staphylococcus aureus inactivated with formalin and the cCHP nanogel, and administered the complex to the mucosa in the nasal cavity of cattle. As a result, it was revealed that unlike the above-described previous report, in the milk of the cattle, that is, in the mammary gland (udder) which secretes the milk, the titer of IgA antibody against Staphylococcus aureus was enhanced.

In addition, it was found that in the case where Staphylococcus aureus was inoculated to the udder of the nasally immunized cattle, this inoculation increased the titer of IgA antibody against Staphylococcus aureus to suppress the proliferation of Staphylococcus aureus. That is, it was revealed that it is possible to prevent bovine mastitis through suppression of the proliferation of Staphylococcus aureus in the udder by making sensitization with a mucosal vaccine composition containing a complex of an antigen derived from Staphylococcus aureus and a cationic nanogel.

Moreover, it was also found that the titer of IgA antibody against ClfA protein of Staphylococcus aureus increases immediately after the inoculation. ClfA protein is one of Staphylococcus aureus membrane proteins, and is known to bind to Annexin A2 protein on the surfaces of the bovine mammary gland epithelial cells to mediate the entry process of Staphylococcus aureus into the epithelial cell (see Ashraf S. et al, Sci Rep., January 19, 2017; 7: 40608). In addition, it is also known that when Staphylococcus aureus enters the mammary gland epithelial cells in the early stage of infection in the bovine mastitis, the bacterium consequently becomes unlikely to immunologically attacked by the antibody (see Almeida RA. et al., J Dairy Sci., 1996, 79(6): pp. 1021 to 1026).

Hence, in a cattle sensitized with a mucosal vaccine composition containing a complex of an antigen derived from Staphylococcus aureus and a cationic nanogel, once Staphylococcus aureus comes into contact with the bovine, a high-titer antibody against ClfA protein is started to be produced, making it possible to suppress the entry of the bacterium into the mammary gland epithelial cells.

As described above, the present inventors found that the above-described vaccine composition makes it possible to effectively protect cattle from infection of Staphylococcus aureus to prevent mastitis, and eventually completed the present invention.

Therefore, the present invention relates to a mucosal vaccine composition for preventing bovine mastitis and a bovine mastitis prevention method using the composition, and more specifically provides the following:
<1> A mucosal vaccine composition for preventing bovine mastitis, comprising:
   an antigen derived from Staphylococcus aureus; and
   a nanogel comprising a polysaccharide in which a hydrophobic functional group and a cationic functional group are added as side chains.
<2> The mucosal vaccine composition according to <1>, wherein
   the hydrophobic functional group is a cholesteryl group.
<3> The mucosal vaccine composition according to <1> or <2>, wherein
   the polysaccharide is pullulan.
<4> The mucosal vaccine composition according to any one of <1> to <3>, wherein
   the cationic functional group is an amino group.
<5> The mucosal vaccine composition according to any one of <1> to <4>, for nasally administering the antigen derived from Staphylococcus aureus.
<6> A method for preventing bovine mastitis, the method comprising the step of:
   administering the mucosal vaccine composition according to any one of <1> to <4> to mucosa of cattle.
<7> A method for preventing bovine mastitis, the method comprising the step of:
   administering the mucosal vaccine composition according to any one of <1> to <5> to mucosa in the nasal cavity of cattle.
<8> The method according to <7>, wherein
   the mucosa in the nasal cavity to which the nasal vaccine composition is administered is the adenoid located deep in the nasal cavity.

### [Advantageous Effects of Invention]

According to the mucosal vaccine composition of the present invention, it is possible to enhance the titer of antibodies (IgA and the like) against Staphylococcus aureus immediately after the contact with the bacteria in the udder. The antibodies whose titers are enhanced include an antibody against the ClfA protein of Staphylococcus aureus, which is involved in the entry into bovine mammary gland epithelial cells. Therefore, the present invention makes it possible to provide a vaccine composition that can prevent bovine mastitis in an early stage and effectively.

### [Brief Description of Drawings]

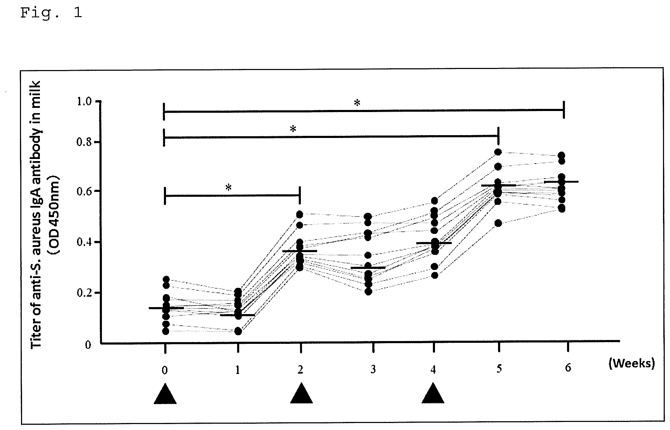

[Fig. 1] Fig. 1 is a graph showing transitions of IgA antibody titers against Staphylococcus aureus (S. aureus) in milks after nasal immunization using a complex of formalin-killed Staphylococcus aureus (FKSA) and a cationic nanogel. Points (black circles) in each data indicate the antibody titers in milks drawn from each nasally immunized cow (milks drawn from 12 udders of 3 cows, n = 12). Black triangles added to the horizontal axis indicate the timings of nasal immunization. Asterisks (*) indicate that significant difference was observed between average values of the respective groups at day 0 and 1 to 6 weeks after the immunization (P<0.05).

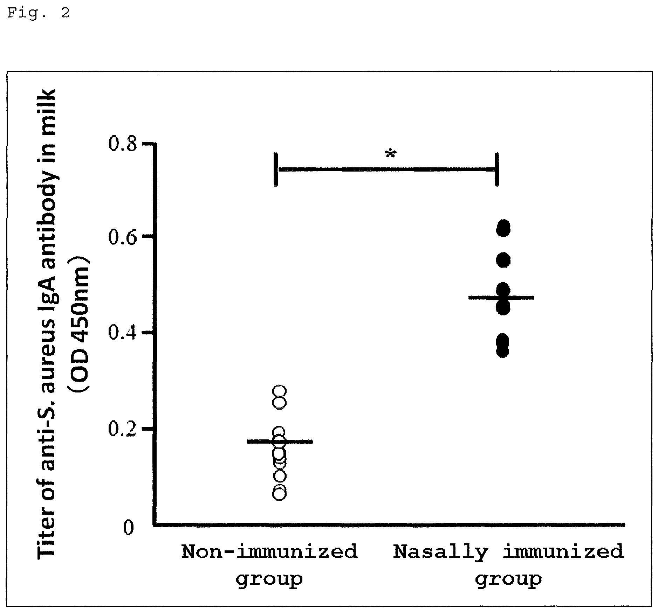

[Fig. 2] Fig. 2 is a graph showing a result of comparing the IgA antibody titers against Staphylococcus aureus in milks between nasally immunized cows and non-immunized cows. In the graph, black circles indicate antibody titers in milks drawn from the nasally immunized cows (12 udders of 3 cows, n = 12). White circles indicate antibody titers in milks drawn from the non-immunized cows (12 udders of 3 cows, n = 12). Bars indicate average values. Asterisk (*) indicates that significant difference was observed between groups (P<0.05).

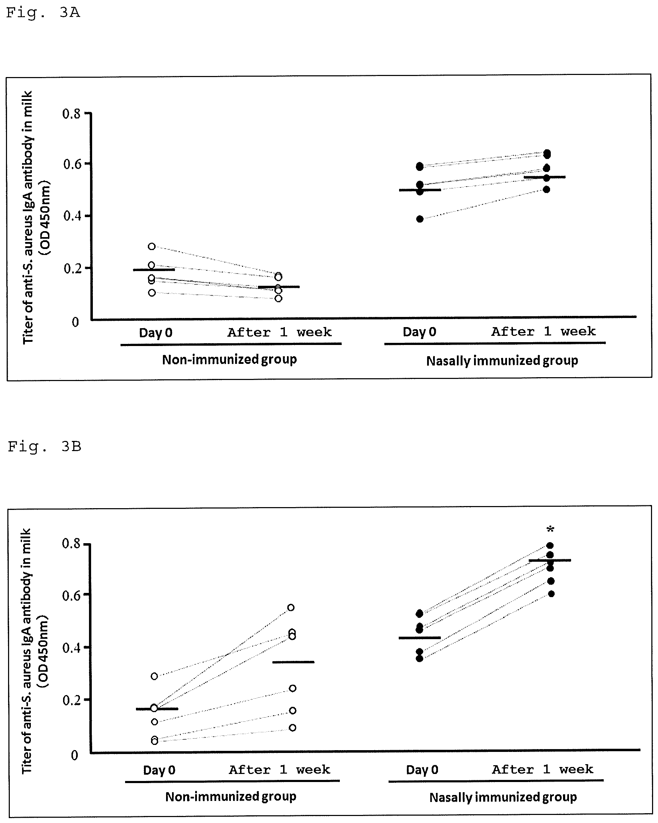

[Fig. 3A] Fig. 3A is a graph showing a result of analyzing IgA antibody titers against Staphylococcus aureus in milks of cows into the udders of which a mock (PBS) was infused. In the graph, black circles indicate antibody titers in milks drawn from the nasally immunized cows (6 udders of 3 cows, n = 6). White circles indicate antibody titers in milks drawn from the non-immunized cows (6 udders of 3 cows, n = 6) . Bars indicate average values. "day 0" and "after 1 week" indicate the timing before the infusion of PBS and at 1 week after the infusion of PBS.
[Fig. 3B] Fig. 3B is a graph showing a result of analyzing IgA antibody titers against Staphylococcus aureus in milks of cows into the udders of which Staphylococcus aureus was infused. In the graph, black circles indicate antibody titers in milks drawn from the nasally immunized cows (6 udders of 3 cows, n = 6). White circles indicate antibody titers in milks drawn from the non-immunized cows (6 udders of 3 cows, n = 6). Bars indicate average values. "day 0" and "after 1 week" indicate the timing before the infusion of Staphylococcus aureus and at 1 week after the infusion of Staphylococcus aureus. Asterisk (*) indicates that significant difference was observed between day 0 and 1 week after the infusion (P<0.05)

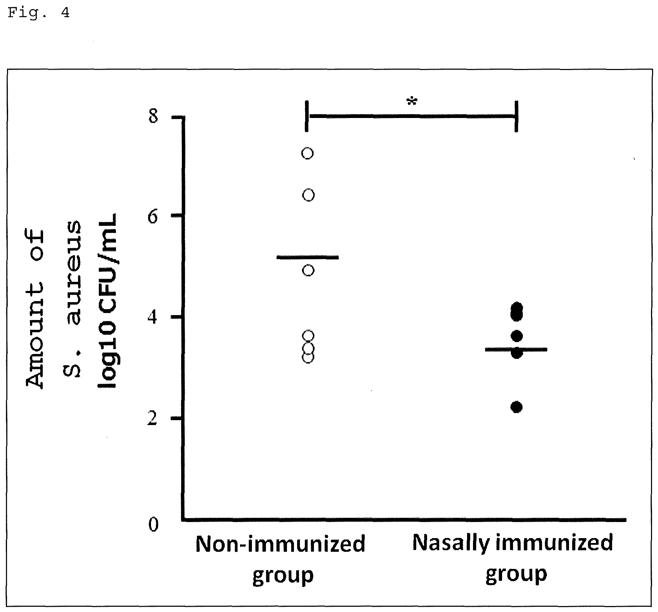

[Fig. 4] Fig. 4 is a graph showing a result of comparing the amounts of Staphylococcus aureus in milks of cows at 1 week after the infusion of Staphylococcus aureus into the udders. In the graph, black circles indicate the amounts of bacterium in milks drawn from the nasally immunized cows (6 udders of 3 cows, n = 6). White circles indicate the amounts of bacterium in milks drawn from the non-immunized cows (6 udders of 3 cows, n = 6). Bars indicate average values. Asterisk (*) indicates that significant difference was observed between groups (P<0.05).

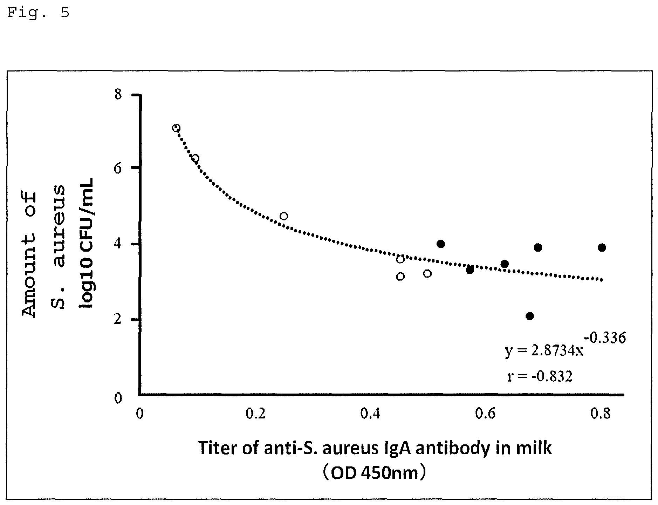

[Fig. 5] Fig. 5 is a graph showing the correlations between the amount of Staphylococcus aureus and the titers of IgA antibody against the bacterium, in milks of cows 1 week after the injection of Staphylococcus aureus into the udder. In the graph, black circles indicate data on milks drawn from the nasally immunized cows (6 udders of 3 cows, n = 6). White circles indicate data on milks drawn from the non-immunized cows (6 udders of 3 cows, n = 6). The statistical correlations were determined by non-linear regression analysis, and P<0.01 was deemed as statistically very significant.

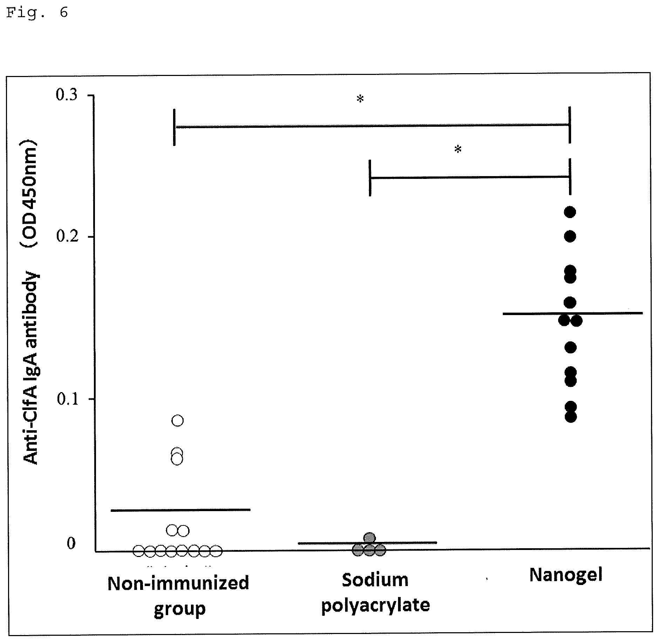

[Fig. 6] Fig. 6 is a graph showing the titers of IgA antibody against ClfA protein in milks of cows after nasal immunization using a complex of FKSA and the cationic nanogel or sodium polyacrylate (after 3 administrations, 6 weeks after the initial immunization). In the graph, black circles indicate antibody titers in milks drawn from the cows nasally immunized using the complex of FKSA and the cationic nanogel (12 udders of 3 cows, n = 12). Gray circles indicate antibody titers in milks drawn from the cow nasally immunized using the complex of FKSA and sodium polyacrylate (4 udders of 1 cow, n = 4). White circles indicate antibody titers in milks drawn from the non-immunized cows (12 udders of 3 cows, n = 12). Bars indicate average values. Asterisks (*) indicate that significant difference was observed between groups (P<0.05) .

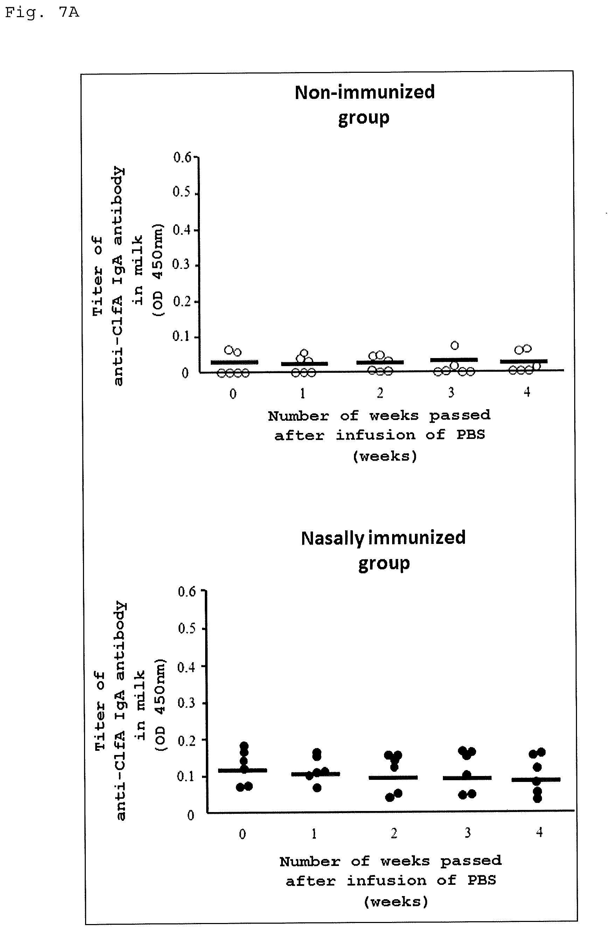

[Fig. 7A] Fig. 7A is a graph showing transitions of IgA antibody titers against ClfA in milks of cows into the udders of which a mock (PBS) was infused. In the graph, black circles indicate antibody titers in milks drawn from the cows nasally immunized using the complex of FKSA and the cationic nanogel (6 udders of 3 cows, n = 6). White circles indicate antibody titers in milks drawn from non-immunized cows (6 udders of 3 cows, n = 6). Bars indicate average values. The numerical values on the horizontal axis indicate the number of weeks that had passed after the infusion of PBS (Note that "0" indicates before the infusion of PBS).

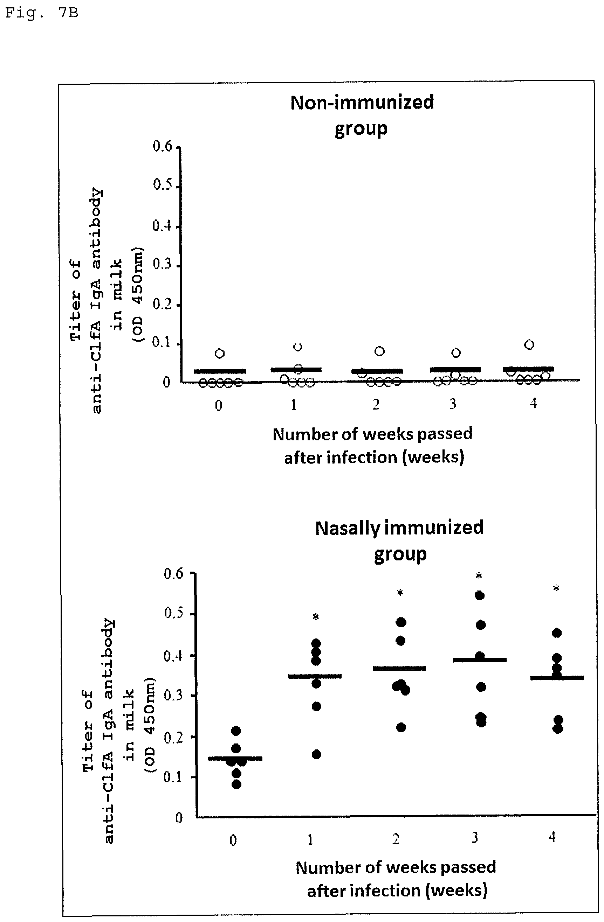

[Fig. 7B] Fig. 7B is a graph showing transitions of IgA antibody titers against ClfA in milks of cows into the udders of which Staphylococcus aureus was infused. In the graph, black circles indicate antibody titers in milks drawn from the cows nasally immunized using the complex of FKSA and the cationic nanogel (6 udders of 3 cows, n = 6). White circles indicate antibody titers in milks drawn from the non-immunized cows (6 udders of 3 cows, n = 6). Bars indicate average values. The numerical values on the horizontal axis indicate the number of weeks that had passed after the infusion of Staphylococcus aureus (Note that "0" indicates before the infusion of Staphylococcus aureus).

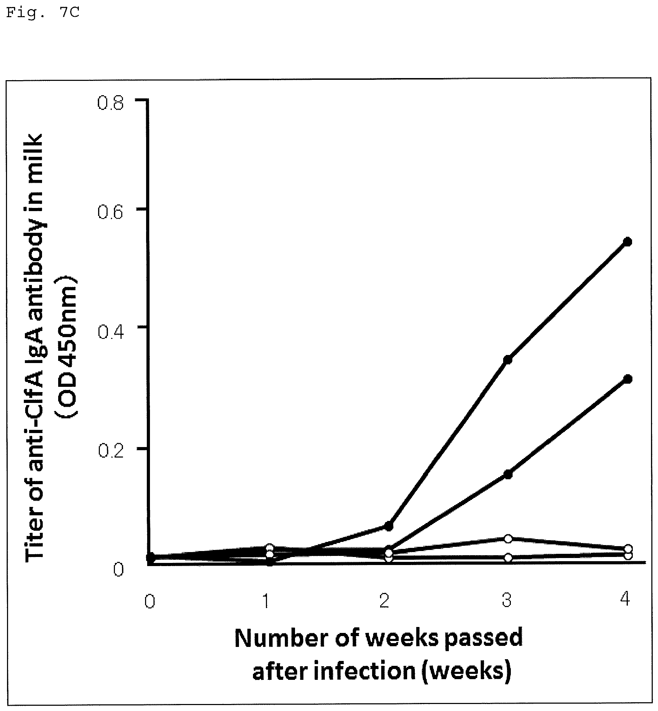

[Fig. 7C] Fig. 7C is a graph showing transitions of IgA antibody titers against ClfA in milk of cows into the udders of which Staphylococcus aureus was infused. In the graph, black circles indicate antibody titers in milks drawn from the cows nasally immunized using the complex of FKSA and sodium polyacrylate. White circle indicate antibody titer in milk of a cow into the udder of which a mock (PBS) was infused (2 udders of 1 cow, n = 2). The numerical values on the horizontal axis indicate the number of weeks that had passed after the infusion of PBS or Staphylococcus aureus (Note that "0" indicates before the infusion of PBS or Staphylococcus aureus).

### [Description of Embodiments]

### (Mucosal Vaccine Composition)

A mucosal vaccine composition of the present invention comprises: an antigen derived from Staphylococcus aureus; and a nanogel comprising a polysaccharide in which a hydrophobic functional group and a cationic functional group are added as side chains.

In the present invention, "Staphylococcus aureus" means bacterium Staphylococcus aureus, also referred to as S. aureus, which is one of facultative anaerobic gram-positive bacteria (staphylococcus) and which can enter and colonize in the bovine mammary gland to induce inflammatory reaction through proliferation. The "Staphylococcus aureus" used in the present invention may be a commercially available strain, or any of strains stored in various research institutions (for example, BM1006 strain, SA003 strain, and JE2 strain), or may be Staphylococcus aureus separated from milk of cattle contracted mastitis.

In the present invention, an "antigen derived from Staphylococcus aureus", which is an active ingredient of the mucosal vaccine composition, only has to have a characteristic of inducing the production of an antibody against the bacterium (so-called immunogenicity) when administered to the mucosa of cattle, and is not particularly limited. However, from the viewpoint that in the case of the viable bacterium, the nanogel covering the bacterium, which will be described later, is likely to be lost along with the proliferation of the bacterium and that side effects due to the vaccination are unlikely to occur, inactivated Staphylococcus aureus or part thereof is preferably used as the antigen according to the present invention.

Inactivated Staphylococcus aureus can be prepared as appropriate by using a publicly-known method such as a formalin treatment, a phenol treatment, a heating treatment, or an ultraviolet ray irradiation treatment, for example. In addition, the "part" of inactivated Staphylococcus aureus is not particularly limited as long as the part has immunogenicity, and may be a fragment of the bacterium, which is obtained by subjecting Staphylococcus aureus to a physical treatment such as sonication, or a degradation product of the bacterium, which is obtained by subjecting Staphylococcus aureus to an enzyme treatment using a hydrolase (lysostaphin or the like), for example. In addition, as long as having immunogenicity, proteins, polypeptides, sugars, glycoproteins, lipids, nucleic acids, and the like of Staphylococcus aureus can be used as the antigen. The proteins and the like may be those isolated and purified from Staphylococcus aureus, or those artificially synthesized (for example, by chemical synthesis).

The mucosal vaccine composition of the present invention comprises, besides the above-described antigen, a cationic nanogel as a delivery system for allowing the antigen to pass through the surface of the negatively charged mucosa (epithelial cells) to deliver the antigen to an immune system such as dendritic cells. The cationic nanogel according to the present invention comprisesa polysaccharide in which a hydrophobic functional group and a cationic functional group are added as side chains as described above.

The "polysaccharide" that serves as a main chain in making the cationic nanogel may be one in which two molecules or more of one or more monosaccharide molecules such as glucose, galactose, mannose, and fructose are bound. Moreover, the polysaccharide may have a modification group such as a hydroxymethyl, hydroxyethyl, hydroxypropyl, or carboxymethyl group. Specific examples of the polysaccharide include pullulan (a homopolysaccharide consisit of α-glucose), amylopectin, amylose, dextran, hydroxyethyl dextran, mannan, levan, inulin, chitin, chitosan, xyloglucan, and water-soluble cellulose. From the viewpoint of their chemical structures and physical properties, pullulan, amylopectin, amylose, dextran, and hydroxyethyl dextran are preferable, and pullulan is more preferable.

In addition, the molecular weight (weight-average molecular weight) of the polysaccharide is generally 10000 to 500000 g/mol, and preferably 30000 to 200000 g/mol. Note that the weight-average molecular weight of the polysaccharide can be determined, for example, by size-exclusion chromatography.

In the cationic nanogel, the "hydrophobic functional group" added as a side chain of the polysaccharide includes, for example, a hydrocarbon group and a steryl group having 8 to 50 carbon atoms (preferably, 12 to 50 carbon atoms); however, from the viewpoint of the hydrophobicity of the functional group and the safety as a food additive, the hydrophobic functional group is preferably a steryl group, and more preferably a cholesteryl group.

The "cationic functional group" added as a side chain of the polysaccharide includes, for example, an amino group, a monoalkylamino group, a dialkylamino group, an imino group, an ammonium group, a guanidino group, an amidino group, cationic amino acid residues (lysine, arginine, histidine and the like); however, from the viewpoint of the synthesis and physical properties, the cationic functional group is preferably an amino group, a monoalkylamino group, or a dialkylamino group, and more preferably an amino group.

The bond between the polysaccharide and the hydrophobic functional group or the cationic functional group may be a direct bond or an indirect bond through a linker group. The linker group includes an ester bond (-COO- or -O-CO-), an ether group (-O-), an amide group (-CONH- or -NHCO-), and a urethane bond (-NHCOO-or -OCONH-). One or a plurality of these may be combined, and an alkylene group, arylene group, aralkylene group having 1 to 10 carbon atoms (a benzylene group, a phenethylene group, or the like) may be further combined as necessary.

The number of hydrophobic functional groups added to the polysaccharide may be adjusted as appropriate depending on the form, size, amount, and the like of the antigen to be captured. However, if the number of hydrophobic functional groups to be added is increased, the solubility to water decreases, and it become difficult to control the particle size of the nanogel, making the dispersion stability be likely to decrease. Thus, the number of hydrophobic functional groups is generally 1 to 10, preferably 1 to several (for example, 5, 4, 3, or 2), and more preferably 1 to 2, per 100 monosaccharides. In addition, the number of cationic functional groups added to the polysaccharide may be adjusted as appropriate depending on the form, size, amount, and the like of the antigen to be captured. However, from the viewpoint that it is easy to obtain sufficient ionic interaction with the surfaces of cells of a mammal, the number of cationic functional groups is generally 1 to 50, preferably 10 to 40, and more preferably 15 to 25, per 100 monosaccharides.

In addition, the size (average particle size) of the "cationic nanogel" comprising a polysaccharide in which a hydrophobic functional group and a cationic functional group are added is generally 5 to 200 nm. However, if the size is increased, it becomes easy for an antigen to form an aggregate and it becomes difficult to uniformly cover the surfaces of the antigens with the nanogel, consequently making the dispersion stability be likely to decrease. Thus, the size is preferably 10 to 100 nm, and more preferably 30 to 50 nm. Note that the average particle size (diameter) can be measured, for example, by dynamic light scattering (DLS) .

The "cationic nanogel" of the present invention can be produced, for example, by preparing a nanogel (hydrophobized polysaccharide nanogel) comprising a polysaccharide in which a hydrophobic functional group is added as a side chain, and introducing a cationic functional group into the nanogel.

More specifically, first, a hydroxyl group-containing hydrocarbon or sterol having 12 to 50 carbon atoms is reacted with a diisocyanate compound represented by OCN-R₁NCO (in the formula, R₁ is a hydrocarbon group having 1 to 50 carbon atoms) to produce an isocyanate group-containing hydrophobic compound reacted with one molecule of the hydroxyl group-containing hydrocarbon or sterol having 12 to 50 carbon atoms. The isocyanate group-containing hydrophobic compound thus obtained and a polysaccharide are reacted to produce a hydrophobic group-containing polysaccharide containing a hydrocarbon group or steryl group having 12 to 50 carbon atoms. Next, a hydrophobized polysaccharide nanogel with a high purity can be produced by purifying the product thus obtained with a ketone-based solvent (see International Publication No. WO2000/12564). In addition, since hydrophobized polysaccharide nanogels are commercially available (for example, cholesteryl pullulan manufactured by NOF Corporation), it is possible to use such a hydrophobized polysaccharide nanogel.

Next, the hydrophobized polysaccharide nanogel dried under reduced pressure is dissolved in a solvent such as dimethyl sulfoxide, and 1,1'-carbonyldiimidazole is added to the solution under a nitrogen gas stream, followed by reacting for several hours (for example, approximately 1 hour) at 20 to 40°C. To the reaction solution, a compound containing a cationic functional group (for example, ethylenediamine) is gradually added, followed by agitating for several hours to several tens of hours (for example, approximately 24 hours). The reaction solution thus obtained is dialyzed against distilled water for several days. A white solid (cationic nanogel) can be produced by freeze-drying the reaction solution after the dialysis (see Ayame H. et al., Bioconjug. Chem., 2008, 19: pp. 882 to 890, PTL 2, International Publication No. WO2014/054588). In addition, the degree of substitution of ethylenediamine and the like can be evaluated by using elemental analysis, H-NMR, or the like.

In the mucosal vaccine composition of the present invention, the antigen is captured (covered or the like) by the cationic nanogel to form a complex. The complex is formed, for example, by mixing the antigen and the cationic nanogel in a buffer and leaving the mixture to stand at 4 to 50°C for 30 minutes to 48 hours. The buffer is not particularly limited and may be adjusted as appropriate depending on the types of the antigen and the cationic nanogel used, but includes, for example, a Tris-HCl buffer solution (50 mM, pH 7.6) and PBS. The mixing ratio of the antigen and the cationic nanogel may be adjusted as appropriate depending on the types of the antigen and the cationic nanogel used. For example, in the case where inactivated Staphylococcus aureus is used as the antigen, the ratio (the number of bacterial cells of Staphylococcus aureus (CFU):cationic nanogel (mg)) is preferably 1 x 10¹⁰ CFU:0.01 mg to 1 x 10¹⁰ CFU:10 mg, and more preferably 1 x 10¹⁰ CFU:0.05 mg to 1 x 10¹⁰ CFU:2 mg. In addition, it is possible to analyze the physicochemical properties of the complex thus prepared by using publicly-known methods. For example, the physicochemical properties can be analyzed by using fluorescence resonance energy transfer (FRET), dynamic light scattering, and zeta potential measurement.

The mucosal vaccine composition of the present invention only has to contain at least the antigen and the cationic nanogel, but may contain other components. The "other components" include pharmaceutically acceptable, publicly-known diluents, stabilizers, preservatives, and antioxidants. The diluents include PBS, Tris-HCl buffer solution, saline, and the like. The stabilizers include gelatin, dextran, sorbitol, and the like. The preservatives include thimerosal, β-propiolactone, and the like. The antioxidants include α-tocopherol and the like. In addition, from the viewpoint of enhancing immunogenicity, the mucosal vaccine composition may contain an adjuvant (an emulsion of an inorganic substance such as an aluminum gel adjuvant, a microorganism or a substance derived from a microorganism (BCG, muramyl dipeptide, Bordetella pertussis, pertussis toxin, cholera toxin, or the like), a surface-active substance (saponin, deoxycholic acid, or the like), an oily substance (a mineral oil, a vegetable oil, an animal oil, or the like), or the like).

### (Method for Preventing Bovine Mastitis)

The present invention provides a method for preventing bovine mastitis, comprising: the step of administering the above-described mucosal vaccine composition to mucosa of cattle.

The "cattle" for which the method of the present invention is used is preferably cows, and includes, for example, the Holstein breed, the Guernsey breed, the Jersey breed, the Ayrshire breed, and the Brown Swiss breed. In addition, the "mastitis" only has to be an inflammation that occurs when the mammary gland is infected by Staphylococcus aureus, and may be subclinical mastitis or clinical mastitis.

The "preventing" bovine mastitis in the present invention includes not only inhibiting the onset of bovine mastitis but also inhibiting the progress of bovine mastitis (inhibiting bovine mastitis from becoming severe).

In the method of the present invention, the "mucosa" to which the mucosal vaccine composition is administered only has to be capable of inducing cells or the like that produce IgA antibody against Staphylococcus aureus and allowing the cells to propagate to the mucosal tissue of the mammary gland when the composition is administered (only has to be a mucosal tissue that functions as an inductive site in a so-called common mucosal immune system (CMIS)), and includes, for example, mucosa in the nasal cavity, mucosa in the oral cavity, mucosa in the bowel, mucosa in the throat, mucosa in the aural cavity, mucosa in the conjunctival sac, mucosa in the vagina, and mucosa in the anus. Among these, mucosa in the nasal cavity is preferable from the viewpoint that influences of digestive enzymes and the like are low and it is easy to induce immune reaction stably. Moreover, the adenoid located deep in the nasal cavity is more preferable from the viewpoint that the mucosal vaccine composition is more likely to be taken in.

The method for "administration" of the mucosal vaccine composition to mucosa is not particularly limited, and the administration can be conducted by spraying, nebulization, application, instillation, infusion, injection, or the like. In addition, the dosage of the mucosal vaccine composition can be determined as appropriate depending on the type of the antigen, the age in weeks, the body weight, the health status of the administration target, and the like, but only has to be a pharmaceutically effective amount. The pharmaceutically effective amount means an amount of an antigen necessary to induce the immune reaction against the antigen contained in the mucosal vaccine composition, and for example, in the case where the antigen to be administered is the entire Staphylococcus aureus, the antigen may be administered several times in an amount of 1 × 10¹⁰ to 1 × 10¹¹ CFU (preferably 3 × 10¹⁰ to 7 × 10¹⁰ CFU, and more preferably 5 × 10¹⁰ CFU) once or several times a day, and administered 1 to 5 times (preferably 3 times) in total every 1 to 5 weeks (preferably every 2 weeks).

### [Examples]

Hereinafter, the present invention will be described in more detail based on Examples; however, the present invention is not limited to the following Examples. In addition, the present Examples were conducted by using ingredients and methods described below.

### (Cow)

Six primiparous, young female cows (Holstein) which have never suffered from clinical or subclinical mastitis were subjected to the following experiments. One-fourth of milk samples drawn from these cows was subjected to evaluations using criteria described below to confirm that these cows did not contract mastitis.

Criteria for diagnosing bovine mastitis in Japan: in the case where infection of Staphylococcus aureus (S. aureus) is negative and the somatic cell count (SCC)<3 × 10⁵ cells/mL in milk, the cow does not contract mastitis. Note that the number of somatic cells (mainly leucocytes) in milk increases along with the progress of mastitis.

### (Preparation of Inactivated Staphylococcus Aureus)

As the antigen to be administered to cows, Staphylococcus aureus was inactivated with formalin according to the method described in Kiku Y. et al., J Vet Med Sci, 2016, 78: pp. 1505 to 1510 to prepare a formalin-killed Staphylococcus aureus (FKSA). Specifically, Staphylococcus aureus BM1006 strain was suspended in 0.5% formaldehyde-containing phosphate-buffered saline (PBS), and was incubated overnight at room temperature for inactivation. Thereafter, the resultant product was washed with PBS three times.

Note that Staphylococcus aureus BM1006 strain (sequence type 352, clonal complex 97, mastitis-causing type) is originally a bacterium isolated from milk provided by a dairy farm in Japan. In addition, it was revealed that the sequence type of this bacterium causes mastitis from sequence typing on mastitis (see Hata E. et al., J Vet Med Sci, 2010, 72: pp. 647 to 652).

### (Formation of Complex of Inactivated Staphylococcus Aureus and Cationic Nanogel)

As a delivery system for the antigen, a cationic nanogel (cCHP nanogel) comprising cationic polysaccharide cCHP (cationic CHP) was selected. The cCHP nanogel was prepared according to the method described in Ayame H. et al., Bioconjug. Chem., 2008, 19: pp. 882 to 890 and Akiyoshi K. et al., J. Control. Release, 1998, 54: pp. 313 to 320.

Specifically, 1000 mg of CHP (Cholesterol-bearing pullulan)(Mw = about 100000 g/mol, cholesterol substitution rate: 1.2/100 monosaccharide), which was dried under reduced pressure, was dissolved in 200 ml of dimethyl sulfoxide (DMSO). To the resultant product, 300 mg of carbonyldiimidazole (CDI) was added under a nitrogen gas stream, followed by reacting at 35°C for 4 hours. Subsequently, 3300 mg of ethylenediamine was added, followed by agitating and reacting at 35°C for 24 hours to introduce an amino group to a side chain of pullulan in CHP as a cationic functional group. The reaction solution obtained was dialyzed against distilled water for several days, followed by freeze drying to obtain 0.905 g of a white flocculent solid (Mw (a value estimated from the molecular weight of pullulan used in CHP as a raw material) = about 100000 g/mol, yield: 82.3%).

After the cCHP thus synthesized was dissolved in PBS by agitation, the size (average particle size) of the cCHP nanogel in the cCHP nanogel dispersion liquid obtained by sonication was 30 to 40 (32 on average) nm as a result of analysis with a dynamic light scattering instrument. In addition, as a result of analyzing the synthesized cCHP nanogel with ¹H-NMR measurement, it was found that 19 ethylenediamine molecules were introduced.

Note that CHP is made of a compound represented by the following formula (I), and in the formula, Rs each independently represent a hydrogen atom or a functional group represented by the following formula (II). In addition, cCHP is made of a compound represented by the following formula (I), and in the formula, Rs each independently represent a hydrogen atom, -C(=O)NHC₂H₄NH₂, or a functional group represented by the following formula (II).

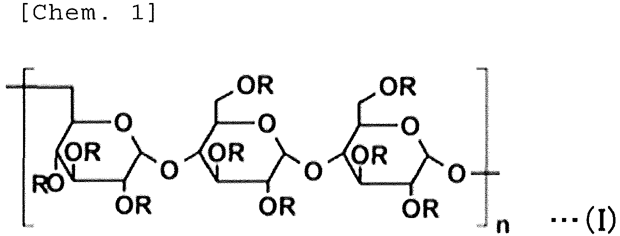

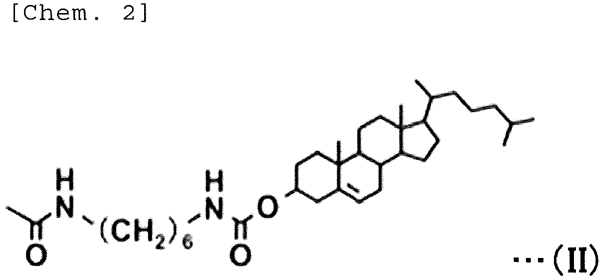

Together with a cCHP nanogel dispersion liquid (nanogel 2 mg) prepared in the same manner as described above, the above-described FKSA (2.5 × 10¹⁰ CFU) was mixed in 2 mL of PBS solution, followed by incubation at 25°C for 30 minutes. Then, the amount was adjusted with PBS to be 1.25 × 10¹⁰ CFU/ mL to prepare an FKSA-captured cCHP nanogel.

Note that for the preparation of the FKSA-captured cCHP nanogel, the conditions that allow the surfaces of FKSA having a particle size of approximately 1 µm to be uniformly covered were studied in advance, and conditions that allow the adsorption (covering) of cCHP nanogel to the surfaces of killed bacterium to reach equilibrium were found and the amount ratio of the FKSA and the cCHP nanogel was determined.

### (Formation of Complex of Inactivated Staphylococcus Aureus and Sodium Polyacrylate)

As the delivery system for the antigen, sodium polyacrylate was also used. For these complexes were the same amount of FKSA as that at the time of adjusting the nanogel was mixed in 5 mL of PBS in which 0.06 g or sodium polyacrylate was dissolved.

### (Nasal Immunization Using Inactivated Staphylococcus Aureus)

The FKSA-captured cCHP nanogel was administered into the nasal cavities of 3 cows three times each with an interval of 2 weeks (2 mL for each administration) to conduct nasal immunization. Note that this administration was conducted by administering the nanogel to the adenoids located deep in the nasal cavities of cows using a syringe equipped with a gavage needle at the front end of the syringe. In addition, in the udder infusion test described below, Staphylococcus aureus was infused into the udder within 4 weeks after the intranasal administration at the third time.

Note that after the nasal immunization, these cows were clinically evaluated in order to judge the health statuses in terms of the local responses (uniformity of the udder, abnormality of milk, appetite, and yield of milk) in the udder as well as the systemic responses (rectal temperature, appetite). As a result, although data is not shown, significant systemic or local responses were not observed.

### (Udder Infusion Experiment of Staphylococcus Aureus)

Staphylococcus aureus BM1006 strain was infused into 6 cows (3 non-immunized cows and 3 nasally immunized cows) in the early stage of lactation. Note that before the infusion of Staphylococcus aureus, a bacteriological test was conducted on the milk samples to confirm that Staphylococcus aureus was completely negative.

The infusion of Staphylococcus aureus into the udders was conducted according to the method described in Nagasawa Y. et al., Anim Sci J., 2018, 89: pp. 259 to 266. Specifically, Staphylococcus aureus was diluted with PBS to have a concentration of about 20 colony-forming units (CFUs) to prepare a Staphylococcus aureus suspension. In addition, after the milking in evening, 2 udders were selected from 4 udders of each cow, the nipples were air-dried, and the Staphylococcus aureus suspension was infused into their mammary gland cisterns. An equivalent amount of PBS was infused into the remaining 2 udders as mocks.

### (Recovery of Milk Samples)

The milk sample was recovered from each udder during milking every morning. The milk sample was recovered every week for 6 weeks after the immunization from day 0 of the immunization (before the immunization was conducted on the day of the nasal immunization). In addition, in the case where the udder infusion experiment of Staphylococcus aureus was conducted, the milk sample was continuously recovered for 1 week from day 0 of the infusion (before the infusion was conducted on the day of the infusion).

### (Analysis of the Amount of Staphylococcus aureus)

The amount of Staphylococcus aureus in each milk sample was analyzed as described below. First, 1 mL aliquot of each milk sample was inoculated on Petrifilm Staphylococcus Aureus Express Count Plate (3M, manufactured by Minneapolis), followed by incubation at 37°C for 24 hours. Then, in the case where the colony took in a color other than reddish violet, the colony was set on Petrifilm Staphylococcus Aureus Express Disk, followed by incubation for 3 hours. After this incubation, the colonies that formed a pink-color zone was counted as Staphylococcus aureus, and CFU/ mL was calculated based on the result of the count.

### (Analysis of Titer of Staphylococcus Aureus-specific IgA Antibody)

To analyze the titer of the Staphylococcus aureus-specific IgA antibody in each milk sample, enzymelinked immunosorbent assay (ELISA) was conducted. Specifically, first, to detect the Staphylococcus aureus-specific IgA antibody, FKSA was coated directly on a microtiter plate as an antigen for capturing. FKSA-containing PBS was inoculated onto a 96-well ELISA plate (C96 Maxisorpcert; Nunc-Immuno Plate, manufactured by Thermo Fisher Scientific) in 5×10⁶ cell/well, followed by drying overnight at 37°C in an oven. After this incubation, wells were washed with Tween 20-containing Tris-buffered saline (TBS-T), followed by incubation with milk at room temperature for 90 minutes. After washing 5 times with TBST, Sheep anti-Bovine IgA Antibody horseradish peroxidase HRP Conjugated (manufactured by Bethyl Laboratories, Inc.) was added to the wells, followed by incubation at room temperature for 2 hours. Subsequently, a newly prepared substrate was added, and the absorbance (OD) at 450 nm was measured using 3',5,5'-tetramethylbenzidine (TMB) Microwell Peroxidase Substrate System (manufactured by KPL).

### (Analysis of Antibody Titer Against ClfA Protein of Staphylococcus Aureus)

As described above, the udder infusion experiment of Staphylococcus aureus was conducted on 3 cows which had been subjected to the nasal immunization using the FKSA-captured cCHP nanogel in advance. In addition, the nasal immunization and the udder infusion experiment were conducted on 1 cow in the same manner as described above except the FKSA-captured cCHP nanogel was changed to a complex of the FKSA and sodium polyacrylate. Note that each dosage of the complex of the FKSA and sodium polyacrylate was 5 mL. Moreover, as control, 3 cows which had not been subjected to the nasal immunization but into the udders of which Staphylococcus aureus was infused as described above.

Then, milk samples were recovered from these cows for each udder according to the above-described method for 4 weeks since Staphylococcus aureus had been infused into the udder. Then, the titer of the antibody against the ClfA protein of Staphylococcus aureus in each milk sample was analyzed using ELISA. Note that this ELISA was conducted in the same manner as in the above-described (Analysis of Titer of Staphylococcus Aureus-specific IgA Antibody) except that the microtiter plate was coated with 5 µg/well ClfA protein in place of FKSA. Note that the ClfA protein was a recombinant ClfA protein (full length) prepared by expressing GST-fusion protein in Escherichia coli (BL21 competent cell) to be extracted and purified, and then further cleaving the GST tag by the GST-fusion protein cleavage protease (manufactured by GE Healthcare, product name: PreScission Protease) treatment.

### (Statistical Analysis)

In the statistical analysis, the amount of Staphylococcus aureus was converted to log¹⁰ such that the amount of Staphylococcus aureus and the antibody titer of Staphylococcus aureus-specific IgA follow normal distribution. The difference between groups was evaluated using Student's t-test. The relevance between the amount of Staphylococcus aureus and the antibody titer was analyzed by applying them to a linear mixed model using SAS software (manufactured by SAS Institute Japan Ltd.). In addition, the relation between the amount of Staphylococcus aureus and the antibody titer was evaluated using Spearman's rank correlation coefficient and non-linear regression analysis. Note that "P<0.05" and "P<0.01" were deemed as "significant" and "very significant", respectively.

Results obtained by using the above-described ingredients and methods are shown below.

### (Example 1)

It was analyzed whether the IgA antibody titer against Staphylococcus aureus was increased in milks of the cows by administering the complex of the inactivated Staphylococcus aureus (FKSA) and the cationic nanogel into the nasal cavity of the cows.

As a result, as shown in Fig. 1, in the milks of the nasally immunized cows (12 udders of 3 cows, n = 12), the anti-Staphylococcus aureus-specific IgA antibody was significantly high at 2 weeks, 5 weeks, and 6 weeks after the immunization as compared with before the immunization (the average absorbances were 0.37 at 2 weeks after the immunization, 0.61 at 2 weeks after the immunization, 0.62 at 5 weeks after the immunization, and 0.17 before the immunization, P<0.05).

In addition, as shown in Fig. 2, the anti-Staphylococcus aureus-specific IgA antibody in the milks sampled from the cows subjected to nasal immunization using FKSA was significantly increased as compared with those of the non-immunized cows (control) (the average absorbances were 0.49 in nasally immunized group vs 0.18 in non-immunized group, P<0.05).

Hence, it was revealed that it is possible to enhance the titer of the IgA antibody against Staphylococcus aureus in milks of cows by administering the complex of the antigen derived from Staphylococcus aureus and the cationic nanogel into the nasal cavities of the cows.

### (Example 2)

Experimental mastitis models were established by inoculating Staphylococcus aureus into the udders after nasally immunized using the complex of FKSA and the cationic nanogel. Then, the anti-Staphylococcus aureus-specific IgA antibody titer and the amount of Staphylococcus aureus in milks sampled from these models were examined.

As a result, first, regarding the anti-Staphylococcus aureus-specific IgA antibody titer, as shown in Fig. 3A, in milks recovered from the udders into which Staphylococcus aureus had not been infused (PBS had been infused as mocks), significant difference at day 0 of the infusion to 1 week after the infusion was not observed in both non-immunized cows and nasally immunized cows (6 udders of 3 cows in each group, each n = 6).

In contrast, as shown in Fig. 3B, in the case where Staphylococcus aureus had been infused into the udders of nasally immunized cows (6 udders of 3 cows, n = 6), the antibody titer at 1 week after the infusion was significantly increased as compared with that at day 0 (the average absorbances were 0.72 at 1 week after the infusion vs 0.42 at day 0, P<0.05) . On the other hand, in non-immunized cows, significant change of antibody titer was not observed (6 udders of 3 cows, n = 6)

Next, the amount of Staphylococcus aureus in each milk was measured. As a result, as shown in Fig. 4, the proliferation of Staphylococcus aureus due to the infusion into the udders was significantly suppressed in nasally immunized cows as compared with non-immunized cows (6 udders of 3 cows in each group, each n = 6. The average values of the amounts of Staphylococcus aureus (= log¹⁰ CFU/ mL) were 3.7 in nasally immunized group vs 5.2 in non-immunized group. P<0.05) .

Note that in the case where the mock was infused, it was confirmed that Staphylococcus aureus was not detected from milks from both non-immunized cows and nasally immunized cows at 1 week after the infusion (6 udders of 3 cows each, each n = 6).

Moreover, in order to confirm that the above-described suppression of proliferation of Staphylococcus aureus in the milks was caused by the anti-Staphylococcus aureus-specific IgA antibody titer enhanced by the nasal immunization of the complex of the FKSA and the cationic nanogel, the correlation between these 2 numerical values after the infusion of Staphylococcus aureus was analyzed using the Spearman's rank correlation coefficient.

As a result, as shown in Fig. 5, in the udders into which Staphylococcus aureus had been infused, a very significant negative correlation was confirmed between the anti-Staphylococcus aureus-specific IgA antibody titer and the amount of Staphylococcus aureus (12 udders of 6 cows, n = 12, r = - 0.832, P<0.01).

Hence, it was revealed that it is possible to enhance the titer of IgA antibody against Staphylococcus aureus to suppress the proliferation of Staphylococcus aureus in the udders by administering the complex of the antigen derived from Staphylococcus aureus and the cationic nanogel into the nasal cavities of the cows.

### (Example 3)

Next, the effect against Staphylococcus aureus was compared between the case where the cationic nanogel was used as the carrier for the antigen derived from Staphylococcus aureus and the case where another carrier (sodium polyacrylate) was used. The results thus obtained are shown in Figs. 6 to 7C.

Note that in this comparison, in order to evaluate the effect against Staphylococcus aureus, the IgA antibody titer against ClfA protein in milks was detected. ClfA protein is one of Staphylococcus aureus membrane proteins, and is known to bind to Annexin A2 protein on the surfaces of the bovine mammary gland epithelial cells to mediate the entry process of Staphylococcus aureus into the epithelial cell (see Ashraf S. et al, Sci Rep., January 19, 2017; 7: 40608). Hence, if the antibody titer against ClfA protein is high, it can be evaluated that the effect of suppressing the entry of Staphylococcus aureus into mammary gland epithelial cells is high, that is, the effect of protection against the Staphylococcus aureus infection is high.

First, it was analyzed whether the IgA antibody titer against ClfA was increased in milks of cows by administering the complex of the FKSA and the cationic nanogel or sodium polyacrylate into the nasal cavities of the cows.

As a result, as shown in Fig. 6, the anti-ClfA-specific IgA antibody titers in milks of cows (12 udders of 3 cows, n = 12) which had been nasally immunized with FKSA using the cationic nanogel in advance were significantly higher than those of non-immunized cows and cows which had been nasally immunized with FKSA using sodium polyacrylate in advance, after 3 immunizations (6 weeks after the start of the initial immunization).

Next, experimental mastitis models were established by inoculating Staphylococcus aureus into the udders after each nasal immunization. Then, in order to analyze the effect by each nasal immunization, the IgA antibody titers against ClfA in milks sampled from these models were examined.

As a result, as shown in Figs. 7A and 7B, in non-immunized cows, the IgA antibody titers against ClfA protein were not enhanced even when Staphylococcus aureus was infused into the udders as in the case of the antibody titers against Staphylococcus aureus described above.

In contrast, in cows which had been nasally immunized with FKSA using the cationic nanogel in advance, the IgA antibody titers against ClfA protein significantly increased immediately after Staphylococcus aureus had been infused. On the other hand, in cows which had been nasally immunized using sodium polyacrylate in advance, no increase in IgA antibody titer against ClfA protein was observed in about 2 weeks after Staphylococcus aureus had been infused (see Fig. 7C).

In addition, although not shown in data, when clinical symptoms after the infusion of Staphylococcus aureus were compared among the case where the cationic nanogel was used, the case where sodium polyacrylate was used, and the case where no immunization was conducted, cows which had been nasally immunized with FKSA using the cationic nanogel showed the most mild symptoms. In particular, 1 in 4 non-immunized cows contracted acute mastitis and was euthanized; however, there was no onset case in cows nasally immunized using the cationic nanogel.

Hence, it was revealed that in the case where cows are nasally immunized with the antigen derived from Staphylococcus aureus using the cationic nanogel, the IgA antibody titers against ClfA protein increase immediately after the inoculation of Staphylococcus aureus, making it possible to prevent the bacteria from entering the udders, and protect cows against the infection of the bacteria. On the other hand, in the case where other carriers were used, such an increase in antibody titers immediately after the inoculation was not observed, and the effect of protection against the infection of Staphylococcus aureus was inferior to the case of using the cationic nanogel.

### [Industrial Applicability]

As described above, the present invention makes it possible to prevent mastitis. Hence, the present invention is very useful in the dairy industry.

## Claims

1. A mucosal vaccine composition for preventing bovine mastitis, comprising:
an antigen derived from Staphylococcus aureus; and
a nanogel comprising a polysaccharide in which a hydrophobic functional group and a cationic functional group are added as side chains.

2. The mucosal vaccine composition according to claim 1, wherein
the hydrophobic functional group is a cholesteryl group.

3. The mucosal vaccine composition according to claim 1 or 2, wherein
the polysaccharide is pullulan.

4. The mucosal vaccine composition according to any one of claims 1 to 3, wherein
the cationic functional group is an amino group.

5. The mucosal vaccine composition according to any one of claims 1 to 4, for nasally administering the antigen derived from Staphylococcus aureus.

6. A method for preventing bovine mastitis, the method comprising the step of:
administering the mucosal vaccine composition according to any one of claims 1 to 4 to mucosa of cattle.

7. A method for preventing bovine mastitis, the method comprising the step of:
administering the mucosal vaccine composition according to any one of claims 1 to 5 to mucosa in the nasal cavity of cattle.

8. The method according to claim 7, wherein
the mucosa in the nasal cavity to which the nasal vaccine composition is administered is the adenoid located deep in the nasal cavity.
